# EUROPÄISCHE PATENTSCHRIFT

(11) **EP 1 519 701 B1**
(45) Veröffentlichungstag und Bekanntmachung des Hinweises auf die Patenterteilung: **09.12.2009**
(21) Anmeldenummer: 03760526.8
(22) Anmeldetag: 18.06.2003
(51) Int. Cl.: A61H 3/00, A61B 5/00

(54) **VORRICHTUNG ZUR MESSUNG UND ÜBERWACHUNG DER TEILBELASTUNG VON ORTHOPÄDISCHEN UND CHIRURGISCHEN PATIENTEN**
DEVICE FOR MEASURING AND MONITORING THE FRACTIONAL LOAD OF ORTHOPAEDIC AND SURGICAL PATENTS
DISPOSITIF POUR MESURER ET SURVEILLER LA SOLLICITATION PARTIELLE DE PATIENTS EN ORTHOPEDIE ET EN CHIRURGIE

(30) Priorität: 19.06.2002 AT 9252002; 16.12.2002 AT 18702002
(43) Veröffentlichungstag der Anmeldung: 06.04.2005
(73) Patentinhaber: Clar, Heimo, 8042 Graz (AT)
(72) Erfinder: CLAR, Heimo, A-8042 Graz (AT); WINTER, Egon, A-8020 Graz (AT)
(74) Vertreter: Matschnig, Franz
(86) Internationale Anmeldenummer: PCT/AT2003/000173
(87) Internationale Veröffentlichungsnummer: WO 2004/000195

(56) Entgegenhaltungen:
- WO-A-01/36051
- US-A- 5 511 571

## Beschreibung

Die Erfindung betrifft eine Vorrichtung zur Messung und Überwachung der Teilbelastung von orthopädischen und chirurgischen Patienten.

In der Unfallchirurgie und der Orthopädie sind die Mediziner sowohl bei konservativer als auch teils bei operativer Therapie mit dem Umstand konfrontiert, daß der Patient die verletzte Extremität nicht vollbelasten darf, um ein sicheres Heilen mit zufriedenstellendem Ergebnis zu garantieren. Handelt es sich hierbei um eine untere Extremität, muß der Patient mit Krücken teilbelasten.

Eine Gewichtsbelastung ist daher von Bedeutung, da erst ein dabei auf den betroffenen Knochen ausgeübter Druck den Reiz für ein suffizientes Knochenwachstum auslöst. Ohne möglichst frühzeitige Belastung wäre daher ein gutes knöchernes Therapieergebnis fraglich. Ebenso ist es im Sinne der modernen frühfunktionellen Behandlung, einen Patienten möglichst frühzeitig zu mobilisieren.

Der Umstand der Teilbelastung setzt seitens des Patienten viel Mitarbeit und Umsicht voraus: Er muß genauen Therapieschemata folgend sein betroffenes Bein mit je nach Diagnose und Therapie unterschiedlicher Gewichtsbelastung aufsetzen, er muß z.B. das Bein 3 Wochen hindurch mit 10 kg belasten. Dies macht aufgrund des Fehlens einer exakten Gewichtssensorik in der menschlichen Sohle klarerweise Probleme!

Derzeit wird dem Patient mittels Waage eintrainiert zu erkennen, wieviel er auf einer Waage belasten muß um genau die angestrebte Gewichtsbelastung zu erreichen. In der nach therapeutischer Exaktheit ringenden modernen Medizin also eine unexakte und fehleranfällige Lösung mit oft schlechtem Ausgang, die in einer Reoperation enden kann.

Aufgrund der Tatsache, daß an Patienten aller Altersgruppen mit unterschiedlicher Compliance so hohe Anforderungen in Mitarbeit und Sorgfalt gestellt werden, ist die bisherige Lösung, welche nicht für ein solches mannigfaltiges Patientengut gedacht war, obsolet.

Bekannt sind folgende Vorrichtungen:

Die Amerikanische Patentschrift US 5 357 6 96 A (PARRIS JOHN) (25.10.1994) zeigt eine Vorrichtung zum Messen der Kraft (Gewichtskraft), welche beim Stehen oder Laufen auf den Fuß des Patienten ausgeübt wird. Es ist eine integrierte Alarmvorrichtung vorgesehen, welche den Patienten darüber informiert, dass eine vorprogrammierte Schwelle überschritten wird. Diese Alarmvorrichtung besteht aus einem Vibrationsgeber.

Es ist weiters eine Aufnahme- und Analyseeinheit (Auswerteeinheit) vorgesehen.

Die Übertragung der Messergebnisse kann über eine Verkabelung oder über Funk erfolgen.

Die gesamte Vorrichtung ist dazu geeignet, in einen Schuh eingebaut zu werden.

Da die Schwelle programmierbar ist, werden selbverständlich mehrere (d.h. beliebige) Obergrenzen einstellbar sein.

Die französische Offenlegungsschrift FR 2 638 340 A (UNIVERSITE CLERMONT FERRANT) (04.05.1990) zeigt einen Sensor für Krücken oder Prothesen und Orthesen , welcher das Gewicht (die nach unten ausgeübte Kraft) , welches auf ihnen lastet, messen soll. Das erzeugte Signal wird verstärkt und gespeichert. Es ist ein Alarmgeber vorgesehen, welcher akustische, taktile oder optische Signale zur Warnung erzeugt.

Es sind sowohl ein oberer als auch ein unterer Grenzwert für die Belastung vorprogrammierbar. Die Alarmvorrichtungen sind im Handgriff eingebaut.

Die Europäische Offenlegungsschrift EP 1 040 811 A2 (MOLNAR) (4.10.2000) zeigt eine anmeldungsgemäße Krücke, bei welcher die Messung mittels einer Druckdose erfolgt.

Die Amerikanische Patentschrift US 5,511,571 (ADREZIN ET AL.) (30.4.1996) beschreibt ein Verfahren und eine Vorrichtung zur Gangüberwachung, wobei eine Gehhilfe wie ein Gehbock, ein Gehstock oder eine Krücke mit Belastungssensoren versehen werden und die beim Gehen auftretenden Belastungen analysiert werden.

Die WO 01/36051 A2 (ANDANTE MEDICAL DEVICES LTD.) zeigt ein tragbares Belastungsüberwachungssystem, das über Messvorrichtungen, die beispielsweise in einen Schuh eingebracht werden können, die Belastung auf Gliedmaßen eines Patienten messen kann.

An diesen Methoden ist als nachteilig anzumerken:

In der Amerikanischen Patentschrift US 5 357 6 96 A wird die Meßvorrichtung in eine Schuh eingebaut. Dies ist technisch aufwendig (orthopädische Schusterarbeit) und ist zudem bei einer Teilbelastungszeit über mehrere Monate unhygienisch. Aufgrund der Nichtwiederverwendbarkeit der Meßvorrichtung ist diese Methode teuer.

Die französische Offenlegungsschrift FR 2 638 340 A und ihr europäisches Pendant EP 1 040 811 A2 weisen keine Synchronisation der Meßparameter der beiden Krücken auf oder messen nur die Gewichtsbelastung einer Krücke, was zu ungenauen Meßergebnissen führt. Es ist Aufgabe der Erfindung, eine Vorrichtung zu schaffen, die ausreichend genaue Meßergebnisse liefert und mit der ein gutes Therapieergebnis auf für den Patienten unkomplizierte Weise erreicht werden kann.

Diese Aufgabe wird mit einer eingangs erwähnten Vorrichtung gelöst, welche erfindungsgemäß aus zumindest einer Meßvorrichtung zur direkten oder indirekten Bestimmung der Gewichtsbelastung einer unteren Extremität sowie weiters aus zumindest einem Alarmgeber besteht, welcher bei einem Überschreiten einer einstellbaren Obergrenze der Belastung ein Alarmsignal abgibt.

Durch die Ausgabe eines Alarmsignals wird der Patient bei einem Überschreiten einer für die Therapie günstigen Belastung gewarnt, sodass er solche Belastungen zuverlässig vermeiden kann. Die Obergrenze kann dabei für verschiedene Patienten je nach gewünschter bzw. notwendiger Belastung eingestellt werden, sodass sich die erfindungsgemäße Vorrichtung einfach für verschiedene Patienten adaptieren lässt.

Vorteilhaft kann es sein, wenn die zumindest eine Meßvorrichtung mit einer Auswerteeinrichtung gekoppelt ist.

Bei einer ersten Ausführungsform der Erfindung sind die zumindest eine Meßvorrichtung zusammen mit dem zumindest einen Alarmgeber ( der z.B. einen akustischen Alarm oder einen Vibrationsalarm abgibt ) und gegebenenfalls der Auswertevorrichtung in einer oder mehreren Krücken eingebaut. Diese Ausführungsform ist einfach zu realisieren und bietet Kostenvorteile, da die Krücken nach abgeschlossener Therapie an einen anderen Patienten weitergegeben werden können.

Bei anderen Ausführungsformen ist die Meßvorrichtung in einer Schuhsohle, in einer Schuheinlage, oder im "Gehstöckel" eines Gehgipses eingebaut. Dies eignet sich beispielsweise für Personen, bei denen die Verwendung von Krücken nichtnotwendig ist, oder welche ohnehin einen Gips tragen müssen.

Besonders einprägsam für den Patienten ist es, wenn der zumindest eine Alarmgeber dazu eingerichtet ist, bei Überschreiten einer einstellbaren Obergrenze der Belastung einen akustischen Alarm auszugeben.

Weniger auffällig ist es, wenn der zumindest eine Alarmgeber dazu eingerichtet ist, bei Überschreiten oder Unterschreiten einer einstellbaren Grenze für die Teilbelastung einen Vibrationsalarm auszugeben.

Damit die Belastungen auch noch nachträglich von einem Therapeuten oder Arzt nachvollzogen werden können, ist bei einer vorteilhaften Ausführungsform vorgesehen, dass Überschreitungen der einstellbaren Obergrenze und Untergrenze der Teilbelastung aufgezeichnet und in einem nichtflüchtigen elektronischen Speicher gespeichert werden.

Schließlich kann es auch noch vorteilhaft sein, wenn mehrere einstellbare Obergrenzen und Untergrenzen vorgesehen sind, und wenn bei Über.- oder Unterschreiten verschiedener Belastungsgrenzen unterschiedliche Alarmsignale, beispielsweise unterschiedliche akustische Alarme ausgegeben werden. Auf diese Weise kann beispielsweise der Patient davon informiert werden, dass sich die Belastung bereits in einem kritischen Bereich befindet, und er diese keinesfalls mehr fortsetzen soll, oder dass eine Belastung in diesem Bereich über einen längeren Zeitraum für die Therapie nachteilig sein kann.

Im folgenden ist die Erfindung an Hand der Zeichnung näher erläutert. In dieser zeigen
Fig. 1 in einem Diagramm die Belastungsverhältnisse beim Gehen mit Krücken unter Teilbelastung unter idealisierten statischen Verhältnissen
Fig. 2 ein Ausführungsbeispiel einer erfindungsgemäßen Vorrichtung, und

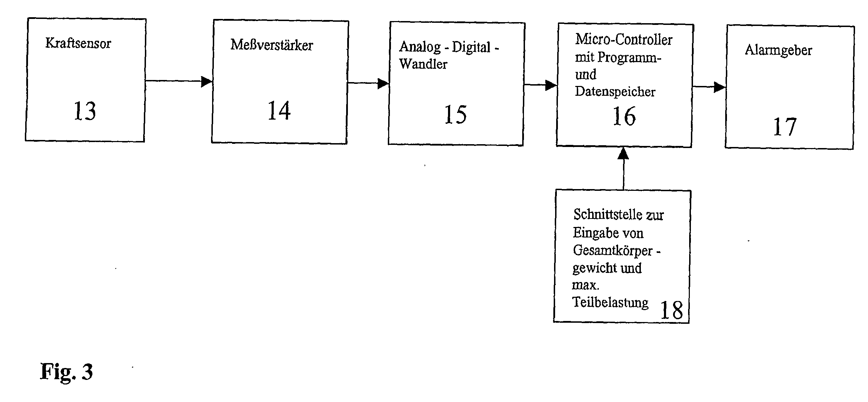

Fig. 3 den Aufbau einer entsprechenden Auswerte-Elektronik.

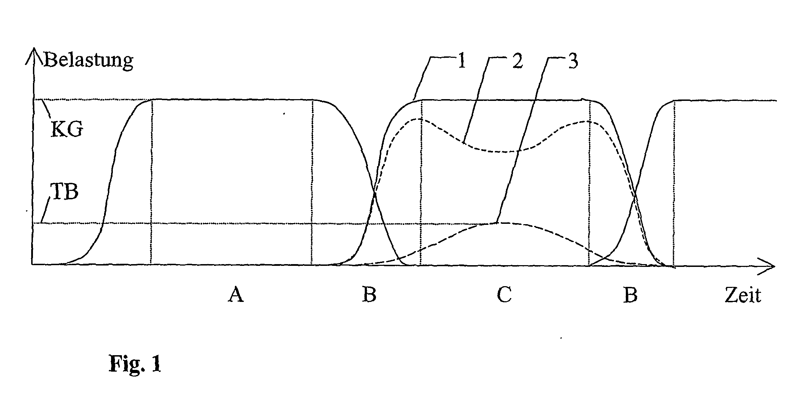

Die Gewichtsbelastung des teilbelasteten Beines wird folgendermaßen ermittelt: : Während des Gehens findet ein fortlaufender Wechsel von Standbein- und Spielbeinphase sowohl für das gesunde wie für das teilzubelastende Bein statt. In der Standbeinphase des gesunden Beines trägt dieses das gesamte Körpergewicht A. In der Standbeinphase des teilzubelastenden Beines wirkt auf dieses Bein und auf die beiden Krücken zusammen eine Belastung 2+3, die dem Gesamtkörpergewicht entspricht 1. Während des Wechsels des einen Beines vom Spielbein zum Standbein und des anderen Beines umgekehrt, findet ein fließender Belastungswechsel statt, welchen wir als Übergangsphase bezeichnen wollen B. Die Fig. 1 beschreibt in einem idealisierten Diagramm die Belastungsverhältnisse beim Gehen mit Krücken unter Teilbelastung.

Abschnitt A zeigt eine Standbeinphase des gesunden Beines. Die Gewichtsbelastung des gesunden Beines erreicht das gesamte Körpergewicht KG, da sich Krücken und das kranke Bein während dieser Phase in der Luft befinden. Die Abschnitte B zeigen den Standbeinwechsel vom gesunden zum kranken Bein und umgekehrt, und Abschnitt C stellt die Standbeinphase des kranken Beines dar. Kurve 1 gibt die Gewichtsbelastung des kranken Beines wieder, wenn keine Krücken verwendet werden. Kurve 2 zeigt bei Verwendung der Krücken die Gewichtsbelastung auf den Krücken alleine. Zu Beginn und Ende der Standbeinphase des teilbelasteten Beines erreicht die Gewichtsbelastung des kranken Beines unter Zuhilfenahme von Krücken jeweils ein Maximum, da das verletzte Bein in seiner Standbeinphase zeitverzögert aufgesetzt und frühzeitig wieder abgehoben wird Kurve 3.

Die tatsächliche Teilbelastung wird dadurch gefunden, indem nach Ablauf der Standbeinphase des teilbelasteten Beines die Höhe des Minimums zwischen den Maxima von Kurve 2 ermittelt wird. Das gesamte Körpergewicht abzüglich des Minimums ergibt die maximale Gewichtsbelastung TB des teilbelasteten Beines in der abgelaufenen Standbeinphase wieder. Sie ergibt sich in Fig.1 als das Maximum von Kurve 3.

Im Folgenden sind nochmals kurz die in Figur 1 verwendeten Bezeichnungen erläutert:
A: Standbeinphase des gesunden Beines
B: Übergangsphase
C: Standbeinphase des teilbelasteten Beines
KG: gesamtes Körpergewicht
TB: maximal ermittelte Teilbelastung
"1": Gesamtbelastung auf dem teibelasteten Bein und den Krücken
"2:" Belastung auf den Krücken alleine
"3": Belastung des teilbelasteten Beines als Differenzkurve von 1 und 2

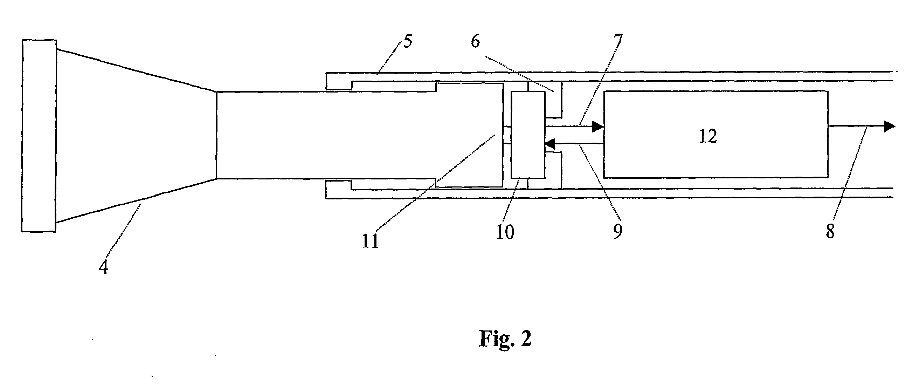

Wie Fig. 2 zeigt, befindet sich im Fußteil (oder an einer anderen geeigneten Stelle) jeder der beiden Krücken ein Kraftaufnehmer 10 wie z. B. eine Kraftmeßdose, ein Membransensor mit Dehnungsmessstreifen, ein direkt an der Krücke außen angebrachter Dehnungsmessstreifen o.ä., welche die tatsächliche Gewichtsbelastung der Krücke mißt. Durch den Kraftaufnehmer wird ein analoges Signal erzeugt, welches proportional zur Gewichtsbelastung der jeweiligen Krücke ist und von der nachfolgenden Schaltung 12 verstärkt und digitalisiert wird.

Dabei erfolgen die Messungen in der einen Krücke mit konstanter Meßrate, die digitalisierten Meßwerte werden mittels Funk zur anderen Krücke übertragen. Für diese Funkübertragung könnte z. B. ein einfaches Telemetriesystem basierend auf ASK (Amplitude Shift Key Modulation) oder FSK (Frequency Shift Key Modulation) mit einer Übertragungsfrequenz in einem der ISM-Frequenzbereiche (Industry, Science, Medicine) verwendet werden, ebenso könnte eine Funkschnittstelle nach dem Bluetooth-Standard zur Anwendung kommen.

Die zweite Krücke empfängt diese Daten, und nach dem Empfang der Daten erfolgt auch in dieser Krücke eine Messung. Die erste Krücke fungiert also als "Master", da in dieser die Messungen ihrer Belastung völlig unabhängig stattfinden, die zweite Krücke hingegen ist in der Rolle des "Slave", da in ihr eine Messung nur stattfindet, wenn sie vorher einen vollständigen Meßwert der ersten Krücke empfangen hat. In der zweiten Krücke (Slave) werden beide Meßwerte addiert, diese Summe der Meßwerte beider Krücken entspricht dem gesamten Körpergewicht abzüglich der Gewichtsbelastung des teilbelasteten Beines - abgesehen von dynamischen Einflüssen durch Abstoß- und Fallbewegungen, die vor allen zu Beginn und am Ende eines Schrittes stattfinden.

Die Summe beider Meßwerte wird in der als Slave fungierenden Krücke laufend von einem Mikrocontroller 16 verarbeitet, der mit Hilfe eines Algorithmus nach dem o.a. Modell die tatsächliche Teilbelastung mit oder ohne Dynamisierung errechnet. Bei Überschreiten der maximal zulässigen Teilbelastung ertönt ein Signalton 17. Anstelle eines akustischen Signalgebers könnte auch ein etwa im Handgriff einer Krücke eingebauter Vibrationsmotor Verwendung finden.

In dieser Krücke kann auch ein Speicher eingebaut sein, der zu jedem Schritt die stattgefundene Teilbelastung speichert oder auch nur die Anzahl der stattgefundenen Überschreitungen der eingestellten Teilbelastungsgrenze. In diesem Fall könnte auch eine Aufschlüsselung z. B. in Anzahl aller Überschreitungen der Teilbelastungsgrenze, in Anzahl der Über.- und Unterschreitungen dieser Grenze z.B. um mehr als 5 Kg, um mehr als 10 Kg usw. stattfinden.

Diese gespeicherten Daten können im nachhinein über Funk oder über Kabelverbindung (z. B. serielle Schnittstelle oder USB) 18 zu einem Computer übertragen und dort weiter verarbeitet werden. Ebenso können die Meßdaten in Echtzeit über Funk (z. B. Bluetooth) zu einem Computer übertragen und dort verarbeitet und extern gespeichert werden. Dies könnte z. B. bei einer Ganganalyse in Echtzeit durch medizinisches Personal im Therapiezimmer zur Anwendung kommen.

In einer zweckmäßigen Ausführung der Meßvorrichtung kann auch eine Untergrenze der Teilbelastung eingegeben werden, bei deren Unterschreiten ebenfalls einer der oben angeführten Alarme gegeben wird, der vom Alarm bei Überschreitungen verschieden ist. Ebenfalls könnte im vorderen Teil des Krückengriffs sichtbar eine Ziffernanzeige (LED oder LCD) eingebaut sein, die eine direkte Anzeige der im jeweils letzten Schritt stattgefundenen Teilbelastung ermöglicht.

Werden die Krücken in einer therapeutisch nicht wünschenswerten Weise verwendet, wie z. B. Laufen, so kann dies von der Auswerteelektronik 12 erkannt werden, und es kann daraufhin ebenfalls ein Alarm gegeben werden sowie eine Speicherung dieser unerwünschten Verwendungsweise erfolgen.

Die Einstellung von Körpergewicht, oberer und unterer Teilbelastungsgrenze kann mit Hilfe eines Computers oder eines anderen externen Gerätes durchgeführt werden, wobei die Übertragung dieser Einstellungen beispielsweise über eine Kabel- oder Funk- oder Infrarotschnittstelle erfolgen kann 18. Ebenso kann an einer der Krücken ein Tastenfeld mit Display angebracht sein, um eine oder mehrere dieser Einstellungen direkt an der Krücke vorzunehmen.

Die Stromversorgung erfolgt z.B. über Batterien, die in oder an der Krücke angebracht sind. Zweckmäßigerweise kann es sich um wiederaufladbare Batterien handeln. Diese können auch in Hohlräumen der Krücke angebracht sein, im geschützten oberen Ende dieses Teiles kann sich ein Stecker für den Anschluß an ein Ladegerät befinden. Um die Krücke mit einer Batterieladung für eine möglichst lange Zeit einsatzfähig zu halten, ist es zweckmäßig, einen Standby-Modus vorzusehen, in den die Schaltung in den Krücken automatisch wechselt, wenn einige Zeit keine Belastung erfolgen sollte, wie z. B. beim Sitzen oder Schlafen. Die Sensoren und die gesamte Elektronik kann im Krückenrohr untergebracht sein, die Krücken müssen daher spritzwasserfest sein, Sensoren und Elektronik müssen unempfindlich gegenüber Temperaturschwankungen sowie Erschütterungen sein.

Eine weitere Möglichkeit der Bestimmung der Teilbelastung ist auch die direkte Messung mit Hilfe von Kraftsensoren oder Drucksensoren, die in einer Einlegesohle oder in einem "Gehstöckel" eines Gehgipses oder direkt in der Schuhsohle untergebracht sind. Die technische Umsetzung gleicht der oben angeführten Methode bis auf den Umstand, daß hier eine Direktmessung der Gewichtsbelastung des betroffenen Beines möglich ist, die Messparameter könnten kabellos zu einer wie oben angeführten Datenverarbeitungsvorrichtung in zumindest einer der beiden Krücken zur weiteren Auswertung übertragen werden.

**Bezugszeichenliste**

| | |
|---|---|
| A. | Standbeinphase des gesunden Beines |
| B. | Übergangsphase |
| C. | Standbeiphase des teilbelasteten oder kranken Beines |
| KG: | Körpergewicht |
| TB: | Maximal ermittelte Teilbelastung |
| 1. | Gesamtbelastung auf dem teilbelasteten Bein und den Krücken |
| 2. | Belastung auf den Krücken alleine |
| 3. | Belastung des teilbelasteten Beines als Differenzkurve von 1 und 2 |
| 4. | Krückenfuß aus Gummi |
| 5. | Krückenrohr |
| 6. | Befestigung der Kraftmeßdose |
| 7. | Analoges Ausgangssignal |
| 8. | Zum Signalgeber |
| 9. | Spannungsversorgung der Kraftmeßdose |
| 10. | Kraftmeßdose |
| 11. | Stempel zur Übertragung der Gewichtsbelastung auf die Kraftmeßdose |
| 12. | Auswerteelektronik (Fig. 3) |
| 13. | Kraftsensor |
| 14. | Meßverstärker |
| 15. | ADC (Analog-Digital-Wandler) |
| 16. | Mikrocontroller mit Programm zur Auswertung der digitalisierten Messungen |
| 17. | Alarmgeber |
| 18. | Schnittstelle zur Eingabe von Körpergewicht und Teilbelastungsgrenze, sowie zum Auslesen der Teilbelastungs-Überschreitungen |

## Patentansprüche

1. Vorrichtung zur Messung und Überwachung der Teilbelastung von orthopädischen und chirurgischen Patienten, bestehend aus zumindest einer Messvorrichtung zur direkten oder indirekten Bestimmung der Gewichtsbelastung zumindest einer unteren Extremität, aus zumindest einem Alarmgeber (17), welcher bei einem Überschreiten oder Unterschreiten einer einstellbaren Belastungsgrenze ein Alarmsignal abgibt, und zumindest einer Auswerteeinheit, sowie aus Krücken
**dadurch gekennzeichnet, dass**
die Auswerteeinheit in einer Krücke eingebaut ist und
Messvornchtungen in beiden Krücken eingebaut sind und die Messung der Teilbelastung in der ersten Krücke (Master) unabhängig von Messungen in der zweiten Krücke (Slave) erfolgt, und eine Messung in der zweiten Krücke nur dann erfolgt, nachdem ein Messwert der ersten Krücke an die zweite Krücke übermittelt wurde.

2. Vorrichtung nach Anspruch 1, **dadurch gekennzeichnet, dass** die zumindest eine Auswerteeinheit und der Alarmgeber (17) in Krücken eingebaut sind.

3. Vorrichtung nach einem der Ansprüche 1 oder 2, **dadurch gekennzeichnet, dass** die Übermittlung der Messwerte mittels Funkübertragung erfolgt.

4. Vorrichtung nach einem der Ansprüche 1 bis 3, **dadurch gekennzeichnet, dass** das Körpergewicht abzüglich der Summe der in den beiden Krücken ermittelten Messwerte der Gewichtsbelastung der unteren Extremität entspricht, wobei durch einen Algorithmus auch die dynamische Gewichtsbelastung ermittelt werden kann.

5. Vorrichtung nach einem der Ansprüche 1 bis 4, **dadurch gekennzeichnet, dass** der zumindest eine Alarmgeber (17) dazu eingerichtet ist, bei Über- oder Unterschreiten einer einstellbaren Belastungsgrenze einen akustischen Alarm auszugeben.

6. Vorrichtung nach einem der Ansprüche 1 bis 5, **dadurch gekennzeichnet, dass** der zumindest eine Alarmgeber (17) dazu eingerichtet ist, bei Über- oder Unterschreiten einer einstellbaren Teilbelastungsgrenze einen Vibrationsalarm auszugeben.

7. Vorrichtung nach einem der Ansprüche 1 bis 6, **dadurch gekennzeichnet, dass** bei Über- oder Unterschreitungen der einstellbaren Belastungsgrenze diese Über- oder Unterschreitungen aufgezeichnet und in einem nichtflüchtigen elektronischen Speicher gespeichert werden.

8. Vorrichtung nach einem der Ansprüche 1 bis 7, **dadurch gekennzeichnet, dass** mehrere einstellbare Ober- und Untergrenzen vorgesehen sind.

## Claims

1. A device for measuring and monitoring the partial weight bearing of orthopaedic and surgical patients, consisting of at least one measuring device for direct or indirect determination of the weight load on at least one lower extremity, at least one alarm signal generator (17) for generating an emergency signal when a measured value exceeds or falls short of a variable weight bearing limit, and at least one evaluation unit, and also crutches
**characterized in that**
said evaluation unit is built into a crutch and measuring devices are built into both crutches and the measurement of the partial weight bearing in the first crutch (master) is carried out irrespective of measurements in the second crutch (slave) and measurement in the second crutch is only carried out when a measured value pertaining to the first crutch has been transferred to the second crutch.

2. The device as defined in claim 1, **characterized in that** said at least one evaluation unit and said alarm signal generator (17) are built into crutches.

3. The device as defined in claim 1 or claim 2, **characterized in that** the transfer of the measured values is carried out by means of radio transmission.

4. The device as defined in any one of claims 1 to 3, **characterized in that** the body weight less the sum of the measured values determined in the two crutches equals the weight load of the lower extremity, whilst the dynamic weight load can be additionally determined by means of an algorithm.

5. The device as defined in any one of claims 1 to 4, **characterized in that** said at least one alarm signal generator (17) is adapted to generate an acoustic alarm when the measured value exceeds or falls short of a variable weight bearing limit.

6. The device as defined in any one of claims 1 to 5, **characterized in that** the at least one alarm signal generator (17) is adapted to generate a vibration alarm signal when the measured value exceeds or falls short of a variable partial weight bearing limit.

7. The device as defined in any one of claims 1 to 6, **characterized in that** when a measured value exceeds or falls short of said variable weight bearing limit the amount of positive or negative deviation therefrom is recorded and stored in a non-volatile electronic memory.

8. The device as defined in any one of claims 1 to 7, **characterized in that** a plurality of variable upper and lower limits is provided.

## Revendications

1. Dispositif pour mesurer et surveiller la sollicitation partielle de patients en orthopédie et en chirurgie, se composant d'au moins un dispositif de mesure pour une détermination directe ou indirecte du poids admissible d'au moins une extrémité inférieure, se composant d'au moins une alarme (17), laquelle émet un signal d'alarme en cas de dépassement au-dessus ou au-dessous d'une limite de charge réglable et se composant d'au moins une unité de contrôle ainsi que de béquilles,
**caractérisé en ce que**
l'unité de contrôle est intégrée dans une béquille, et
**en ce que** des dispositifs de mesure sont intégrés dans les deux béquilles et la mesure de la sollicitation partielle est effectuée dans la première béquille (maître) indépendamment des mesures dans la seconde béquille (esclave), et une mesure est alors effectuée dans la deuxième béquille uniquement après qu'une valeur de mesure de la première béquille a été transmise à la deuxième béquille.

2. Dispositif selon la revendication 1, **caractérisé en ce que** ladite au moins une unité de contrôle et l'alarme (17) sont intégrés dans les béquilles.

3. Dispositif selon l'une des revendications 1 ou 2, **caractérisé en ce que** la transmission des valeurs mesurées est effectuée au moyen d'une radio-transmission.

4. Dispositif selon l'une des revendications 1 à 3, **caractérisé en ce que** le poids déduction faite du total des valeurs mesurées déterminées dans les deux béquilles correspond au poids admissible de l'extrémité inférieure, le poids admissible dynamique pouvant également être déterminé par un algorithme.

5. Dispositif selon l'une des revendications 1 à 4, **caractérisé en ce que** ladite au moins une alarme (17) est réglée pour émettre une alarme acoustique en cas de dépassement au-dessus ou en dessous d'une limite de sollicitation partielle réglable.

6. Dispositif selon l'une des revendications 1 à 5, **caractérisé en ce que** ladite au moins une alarme (17) est réglée pour émettre une alarme par vibrations en cas de dépassement au-dessus ou en dessous d'une limite de sollicitation partielle réglable.

7. Dispositif selon l'une des revendications 1 à 6, **caractérisé en ce que** ces dépassements supérieurs ou inférieurs sont enregistrés en cas de dépassement au-dessus ou en dessous d'une limite de sollicitation réglable et **en ce qu'**ils sont enregistrés dans une mémoire électronique non volatile.

8. Dispositif selon l'une des revendications 1 à 7, **caractérisé en ce que** plusieurs limites inférieure et supérieure réglables sont prévues.
